**Europäisches Patentamt**

**European Patent Office**

**Office européen des brevets**

(19)

(11) Veröffentlichungsnummer : **0 250 853 B1**

(12) # EUROPÄISCHE PATENTSCHRIFT

(45) Veröffentlichungstag der Patentschrift :
**09.01.91 Patentblatt 91/02**

(51) Int. Cl.⁵ : **A61M 16/00, A61M 16/20, A61H 31/00**

(21) Anmeldenummer : **87107500.8**

(22) Anmeldetag : **22.05.87**

(54) Vorrichtung zur Herzmassage und zur Beatmung.

(30) Priorität : **23.05.86 DE 3617327**

(43) Veröffentlichungstag der Anmeldung :
**07.01.88 Patentblatt 88/01**

(45) Bekanntmachung des Hinweises auf die
Patenterteilung :
**09.01.91 Patentblatt 91/02**

(84) Benannte Vertragsstaaten :
**AT BE CH DE ES FR GB GR IT LI LU NL SE**

(56) Entgegenhaltungen :
**DE-B- 2 217 614**
**FR-A- 2 367 484**
**US-A- 2 737 177**
**US-A- 3 105 488**

(56) Entgegenhaltungen :
**US-A- 3 216 413**
**US-A- 3 304 939**
**US-A- 4 297 999**
**US-A- 4 349 015**

(73) Patentinhaber : **Frimberger, Erintrud**
**Josef-Kösel-Weg 10**
**D-8960 Kempten (DE)**

(72) Erfinder : **Frimberger, Eckart, Dr.**
**Tristanstrasse 12**
**D-8000 München 40 (DE)**

(74) Vertreter : **Körber, Wolfhart, Dr.rer.nat. et al**
**Patentanwälte Dipl.-Ing. H. Mitscherlich**
**Dipl.-Ing. K. Gunschmann Dr.rer.nat. W.**
**Körber Dipl.Ing. J. Schmidt-Evers Dipl.-Ing. W.**
**Melzer Steinsdorfstrasse 10**
**D-8000 München 22 (DE)**

EP 0 250 853 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Vorrichtung zur Herzmassage und zur Beatmung mit einem mit Mund und-/oder Nase eines zu beatmenden Patienten verbindbaren Patientenadapter, wie eine Atemmaske oder ein Intubationsschlauch, mit einem auf das Brustbein des zu massierenden Patienten aufzusetzenden komprimierbaren, elastisch rückexpandierenden Beatmungsbeutel, mit einem dem Beatmungsbeutel zugeordneten Ansaugventil, über das Luft in den Beatmungsbeutel bei dessen Expansionsbewegung saugbar ist, und mit einem Patientenventil in einer Verbindungsleitung entsprechender Länge zwischen Beatmungsbeutel und Patientenadapter mit einem verstellbaren Teil wie einer Membran, das bei Ausübung der Kompression den Strömungsweg zwischen Beatmungsbeutel und Patientenadapter freigibt und eine Auslaßöffnung verschließt und das bei Lösen des Kompressionsdruckes den Strömungsweg zwischen Beatmungsbeutel und Patientenadapter verschließt und die Auslaßöffnung zwecks Ausatmens freigibt.

Solche Vorrichtungen werden auch Wiederbelebungs- oder Reanimationsgeräte genannt.

Bei einem plötzlichen Kreislaufstillstand wie bei einem Herzinfarkt, ist es unbedingt erforderlich, sofort mit der Wiederbelebung (Reanimation) zu beginnen. Jede Verzögerung verschlechtert die Erfolgsaussichten u.U. erheblich. Bei der Wiederbelebung ist an dem Patienten sowohl eine Beatmung als auch eine Herzmassage durchzuführen. Die derzeit am weitesten verbreitete Vorgehensweise erfordert zwei Personen. Die eine Person beatmet mit Hilfe eines Beatmungsbeutels, die andere Person führt abwechselnd hierzu eine Herzmassage durch. Bei der Beatmung wird durch Kompression des Beatmungsbeutels Atemluft über den Patientenadapter zwangsweise der Lunge des Patienten zugeführt. Nach der Zufuhr wird durch Auslösen der Expansionsbewegung des Beatmungsbeutels der Ausatemvorgang über das umgeschaltete Patientenventil erreicht. Nach erfolgter Ausatmung drückt die zweite Person das Brustbein des Patienten mit dem Handballen mehrfach nieder und komprimiert dadurch das hinter dem Brustbein liegende Herz.

In der Praxis hat sich gezeigt, daß in den in aller Regel unerwarteten und akuten Notsituationen des Kreislaufstillstandes (Herzstillstandes) häufig nicht sofort, d.h. innerhalb weniger Minuten, zwei geschulte Helfer verfügbar sind. Beispielsweise ist beim Nachtdienst in Kliniken in aller Regel nur ein einziger Arzt anwesend. Auch in Arztpraxen steht meist nur eine geschulte ausgebildete Person zur Verfügung.

Es ist daher erwünscht, beide Vorgänge, Beatmung und Herzmassage, durch eine Person durchführen zu können.

Es ist bereits vorgeschlagen worden, den Beatmungsbeutel so auszubilden, daß er auf das Brustbein des Patienten aufsetzbar ist. Nach der Kompression des Beatmungsbeutels (Beatmungsvorgang) soll bei weiterhin komprimiertem Beatmungsbeutel die Herzmassage dadurch durchgeführt werden, daß der Handballen nicht direkt auf das Brustbein, sondern unter Zwischenlage des komprimierten Beatmungsbeutels einwirkt. Bei einem solchen Einmann-Reanimationsgerät ist jedoch nachteilig, daß nur durch besondere zusätzliche Maßnahmen das Ausatmen des Patienten erzielt werden kann, bevor mit der Herzmassage begonnen wird. Möglichkeiten hierzu sind in der DE-OS 22 17 614 und der DE-OS 22 45 993 angegeben. Wie erwähnt, wird bei üblichen Beatmungsbeuteln der Ausatemvorgang dann ausgelöst, wenn das Patientenventil des Patientenadapters die Auslaßöffnung freigibt. Dies wird bei herkömmlichen Beatmungsbeuteln dann erreicht, wenn durch die Rückexpansion der das verstellbare Teil des Patientenventils gegen die Auslaßöffnung drückende Überdruck beseitigt und damit die Auslaßöffnung freigegeben wird. Um dies zu erreichen, muß zumindest kurzzeitig die Expansionsbewegung des Beatmungsbeutels ausgelöst werden. Ganz offensichtlich wäre hierzu außerordentlich geschultes Personal erforderlich, wobei darüber hinaus bei der Hektik einer Notfallsituation keinesfalls stets die sichere Handhabung gewährleistet werden kann. D.h., daß die Herzmassage häufig in eingeatmetem Zustand, d.h. bei geblähter Lunge durchgeführt würde, was wegen zu hohem Druck nicht nur ungünstig ist, sondern möglicherweise zu einer weiteren Gefährdung des Patienten beitragen könnte. Es ist daher schon versucht worden, eine zwangsweise Ausatmung auszulösen. Gemäß einem der erwähnten Vorschläge ist das Patientenventil konstruktiv so ausgebildet, daß durch Druck auf die Lunge ein Druck gegen das verstellbare Teil des Patientenventils derart ausgeübt wird, daß dieses von der die Auslaßöffnung verschließenden Anlage gelöst wird. Abgesehen davon, daß ein solches Patientenventil kompliziert ausgebildet und daher kostspielig ist, ist nicht sicherstellbar, daß bereits zu Beginn der Herzmassage der Ausatemzustand erreicht ist. Dies insbesondere auch deshalb, weil abgewartet werden muß, bis der Ausatemzustand ausgelöst und tatsächlich erreicht ist.

Ausgehend davon ist es Aufgabe der vorliegenden Erfindung, eine Vorrichtung der eingangs genannten Art so auszubilden, daß im Gebrauch eine Herzmassage sicher im ausgeatmeten Zustand durchgeführt werden kann.

Die Aufgabe wird erfindungsgemäß durch ein normalerweise geschlossenes Entlastungsventil gelöst, das mit dem Beatmungsbeutel oder der Verbindungsleitung zwischen Beatmungsbeutel und Patientenventil oder dem Patientenventil verbunden ist und dessen verstellbares Teil ein Steuerteil aufweist, das so in dem Kompressionsbewegungsweg des Beatmungsbeutels angeordnet ist, daß es bei Beendigung der Kompressionsbewegungsweges das verstellbare Teil zwangsweise in die Offenstellung bewegt zur Lösung des Kompressionsdruckes.

Erfindungsgemäß wird also am Ende des Kompressionsweges, d.h. am Ende des Beatemvorganges zwangsweise der das verstellbare Teil des Patientenventils gegen die Auslaßöffnung drückende Überdruck abgebaut, wodurch zwangsläufig der Ausatemvorgang über die Auslaßöffnung des Patientenventils ausgelöst wird. Die einzige die Vorrichtung bedienende Person muß nicht darauf achten, ob der Ausatemvorgang tatsächlich ausgelöst worden ist, da dieser zwangsläufig ausgelöst wird. Sie kann daher im Anschluß an die Kompression des Beatmungsbeutels sofort mit der Herzmassage beginnen. Bei Beendigung der Herzmassage wird durch Loslassen des Beatmungsbeutels dessen Ansaugvorgang ohne weiteres ausgelöst, an den sich dann der Kompressionsvorgang, d.h. der nächste Atemvorgang anschließen kann. Daraus folgt, daß die erfindungsgemäße Vorrichtung auch von weniger geschulten Personen angewendet werden kann, zumindest dann,wenn der Patientenadapter in Lage gebracht ist und der Beatmungsbeutel auf das Brustbein aufgesetzt ist.

Die Erfindung wird durch die Merkmale der Unteransprüche weitergebildet.

Die Erfindung wird im folgenden anhand der in der Zeichnung schematisch dargestellten verschiedenen Ausführungsbeispielen näher erläutert. Es zeigen :

Fig. 1 schematisch die vier wesentlichen Schritte bei der Wiederbelebung mit Hilfe einer von einer Person bedienbaren Vorrichtung gem. der vorliegenden Erfindung ;
Fig. 2 schematisch im Schnitt einen erfindungsgemäß ausgebildeten Beatmungsbeutel ;
Fig. 3 schematisch eine andere Ausführungsform einer erfindungsgemäß ausgebildeten Vorrichtung ;
Fig. 4 schematisch eine weitere Ausführungsform einer erfindungsgemäß ausgebildeten Vorrichtung ;
Fig. 5 schematisch im Schnitt weitere Einzelheiten eines Beatmungsbeutels für eine Vorrichtung gemäß der Erfindung ;
Fig. 6 schematisch eine Ausbildung einer Führungseinrichtung für einen Beatmungsbeutel ;
Fig. 7 schematisch die Ausbildung einer anderen Führungseinrichtung ;
Fig. 8 schematisch eine Ausführungsform zur Zufuhr von Sauerstoff ;
Fig. 9 eine andere Ausführungsform für die Zufuhr von Sauerstoff zu einer Vorrichtung gemäß der Erfindung.

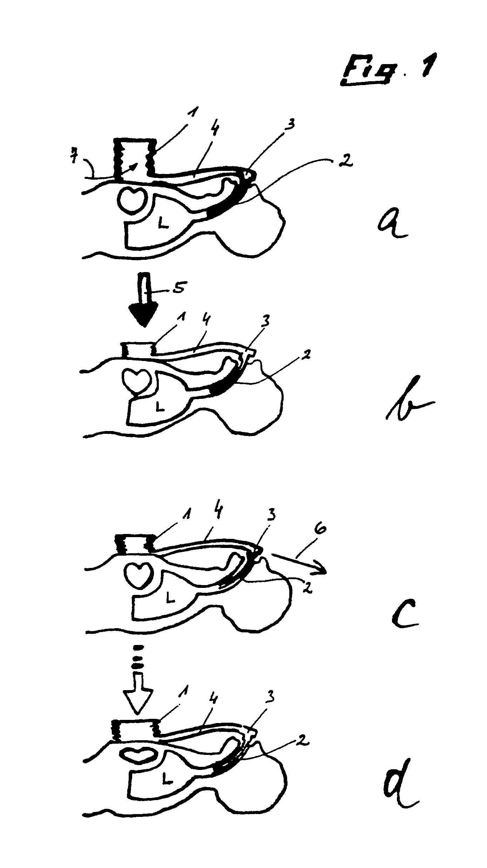

Anhand Fig. 1 wird zunächst der grundsätzliche Aufbau einer erfindungsgemäßen Vorrichtung sowie deren Arbeitsweise näher erläutert.

Die erfindungsgemäße Vorrichtung besteht aus einem Beatmungsbeutel 1, dessen näherer Aufbau weiter unten erläutert wird, einem Patientenadapter 2, der hier als Intubationsschlauch ausgebildet ist, einem Patientenventil 3, dessen grundsätzlicher Aufbau weiter unten erläutert wird,und einer Verbindungsleitung 4 zwischen dem Beatmungsbeutel 1 und dem Patientenventil 3. Wie weiter unten näher erläutert, ist dem Beatmungsbeutel 1 ferner ein Luftansaugventil zugeordnet, das in Fig. 1 nicht näher dargestellt ist. Der Beatmungsbeutel 1 ist komprimierbar und elastisch rück-expandierbar, wobei er bei der Expansion über das erwähnte Luftansaugventil Umgebungsluft ansaugt und den Innenraum des Beatmungsbeutels 1 hiermit füllt.

Im Schritt 1 wird der Patientenadapter dem Patienten zugeführt, d.h., der Intubationsschlauch wird über den Mund des Patienten in dessen Rachen eingeführt. Die Nase des Patienten wird gegebenenfalls durch eine Klammer oder dergleichen (nicht dargestellt) verschlossen. Der Beatmungsbeutel 1 wird auf das Brustbein des Patienten aufgesetzt, unter dem das schematisch dargestellte Herz liegt. Es ergibt sich somit eine Anordnung gemäß der Darstellung nach Fig. 1a. Es ist zu erwähnen, daß der Patient auf einer harten Unterlage flach liegt. Gemäß Fig. 1b wird gemäß dem Pfeil 5 der Beatmungsbeutel 1 komprimiert, wodurch Luft über die Verbindungsleitung 4 zum Patientenventil 3 und von dort über den Patientenadapter 2 in die schematisch dargestellte Lunge L strömt. Der Patient wird beatmet, die Lunge ist gebläht. Durch die erfindungsgemäße Ausbildung der Vorrichtung wird im Schritt Fig. 1c, ohne Expansion des Beatmungsbeutels 1 die Ausatmung der Lunge über das Patietenventil 3 gemäß dem Pfeil 6 erreicht. Die Ausatmung kann dabei gegebenenfalls auch über ein PEEP-Ventil im Patientenventil 3 erfolgen. Anschließend wird gemäß Fig. 1d die Herzmassage durch rhythmisches Komprimieren des unter dem Brustbein liegenden Herzens erreicht, wobei zwischen der die rhythmischen Kompressionsbewegungen des Brustbeins ausübenden Hand und dem Brustbein der Beatmungsbeutel 1 in seinem komprimierten Zustand angeordnet ist. Nach Loslassen des Beatmungsbeutels 1, d.h. nach Beendigung der Herzmassage, expandiert der Beatmungsbeutel 1 in die Stellung gemäß Fig. 1a zurück unter Ansaugen von Luft gemäß dem Pfeil 7 über das hier nicht dargestellte Luftansaugventil. Die Ausgangsstellung gemäß Fig. 1a ist wieder erreicht, der Wiederbelebungsvorgang gemäß den Fig. 1a bis 1b wird zyklisch wiederholt.

Es sei erwähnt, daß an Stelle des Intubationsschlauchs auch eine handelsübliche Atemmaske oder ein anderer Patientenadapter 2 verwendet werden kann.

In den folgenden Fig. sind die gleichen oder entsprechenden Bauelemente wie in Fig. 1 mit den glei-

chen Bezugzeichen versehen.

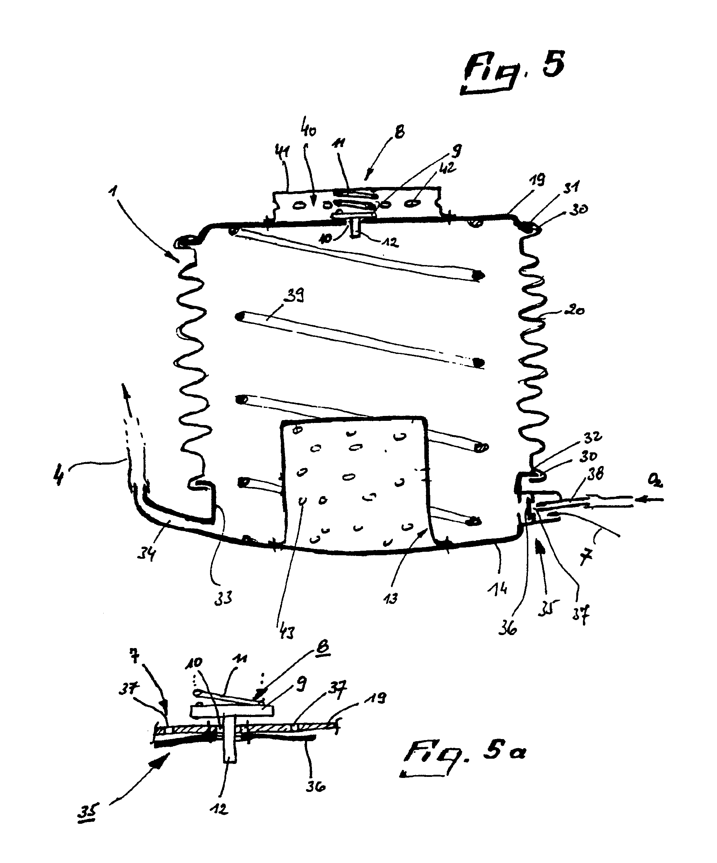

Im folgenden wird auf eine Ausbildung eines Beatmungsbeutels 1 Bezug genommen, die anhand Fig. 5 näher erläutert wird. Jedoch kann auch eine andere Bauweise eines Beatmungsbeutels verwendet werden, sofern die erfindungsgemäß erreichte zwangsweise Entlastung sichergestellt werden kann.

Fig. 2a zeigt den Beatmungsbeutel 1 in expandiertem Zustand. In diesem Zustand ist über ein nicht näher dargestelltes Ansaugventil gemäß dem Pfeil 7 Luft in den Innenraum geströmt. Ein in Fig. 2a dargestelltes Entlastungsventil 8 ist geschlossen, d.h. dessen verstellbares Teil 9 verschließt eine Ausströmöffnung 10 mit Hilfe einer Feder 11. Von dem verstellbaren Teil 9, in der Regel eine Scheibe oder eine Platte, ragt ein Steuerteil 12 in den Innenraum des Beatmungsbeutels 1.

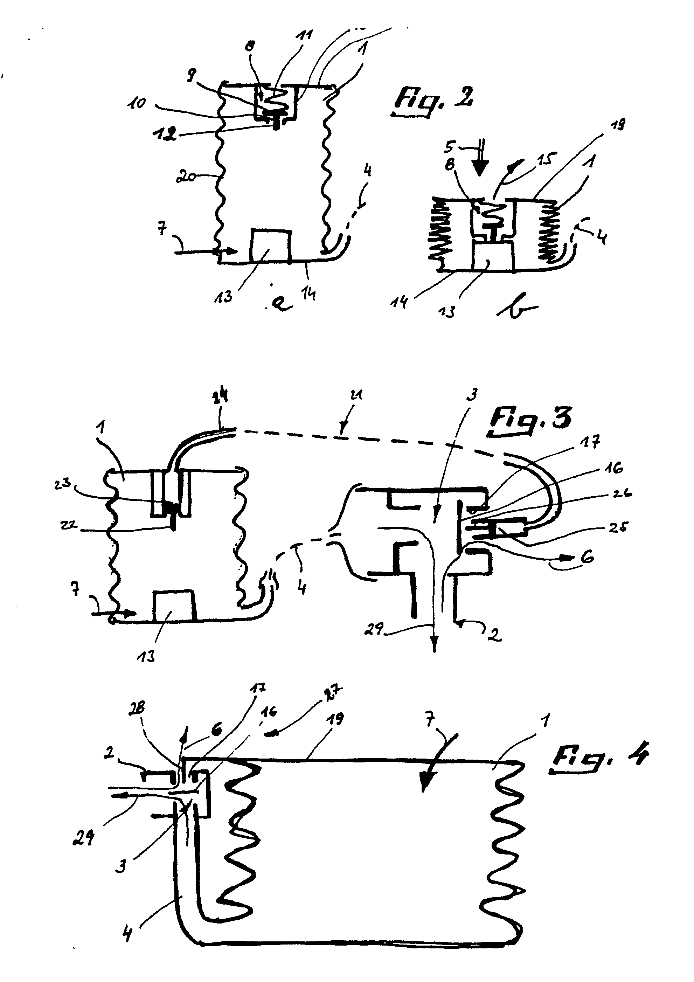

Erfolgt eine Kompression gemäß dem Pfeil 5, wie in Fig. 2b dargestellt, kommt am Ende des Kompressionsweges das Steuerteil 12 zur Anlage gegen ein Widerlager 13 an der Bodenplatte 14 des Beatmungsbeutels 1. Durch die Anlage wird das Steuerteil 12 und damit auch das verstellbare Teil 9 des Entlastungsventils 8 gegen die Kraft der Feder 11 von der Öffnung 10 abgehoben, wodurch Luft aus dem Innenraum des Beatmungsbeutels 1 gemäß dem Pfeil 15 nach außen strömen kann. Auf diese Weise wird jedoch der Überdruck in der Verbindungsleitung 4 und damit auch im Patientenadapter 2 abgebaut. Durch den Druckabbau, vergleiche Fig. 3, wird das verstellbare Teil 16 des Patientenventils 3 von der Ausströmöffnung 17 so abgehoben, daß die Luft praktisch schlagartig aus der Lunge gemäß dem Pfeil 6 durch die Ausströmöffnung 17 ins Freie austreten kann. Es erfolgt somit eine zwangsweise Ausatmung. Bei dem dargestellten Ausführungsbeispiel gemäß Fig. 2 ist das Entlastungsventil 8 in einem Widerlager 18 in einer Deckplatte 19 des Beatmungsbeutels 1 vorgesehen. Der komprimierbare bzw. expandierbare Teil des Beatmungsbeutels 1 ist im wesentlichen durch einen Faltenbalg 20 gebildet, der zwischen Deckplatte 19 und Bodenplatte 14 angeordnet und an diesen dicht befestigt ist. Wie schematisch dargestellt, ist durch konstruktive Ausbildung des Widerlagers 13 oder des Widerlagers 18 bzw. des Entlastungsventils 8 sichergestellt, daß die Druckentlastung über die Öffnung 10 stattfinden kann, wenn die Anlage zwischen dem Entlastungsventil 8 und dem Widerlager 13 erreicht ist. Dies kann durch die Öffnung umgebende Rippen, Noppen, Zähne oder andere Vorsprünge sichergestellt werden.

Da es im wesentlichen darauf ankommt, bei Erreichen des Kompressionsweg-Endes bei der Kompressionsbewegung . des Beatmungsbeutels 1 sicherzustellen, daß das verstellbare Teil 16 des Patientenventils 13 zwangsweise von der Austrittsöffnung 17 abgehoben wird, sind auch andere Ausführungsformen denkbar.

Fig. 3 zeigt eine pneumatisch/hydraulisch arbeitende Anordnung 21. Ein dem Steuerteil 12 entsprechendes Steuerteil 22 eines verschiebbaren Kolbens 23 wird durch die Anlage gegen das Widerlager 13 in Fig. 3 nach oben verschoben. Dadurch wird ein nicht kompressibles Fluid in einer Verbindungsleitung 24 verschoben, die ihrerseits einen weiteren Kolben 25 verschiebt, von dem ein Steuerteil 26 in Anlage gegen das verstellbare Teil 16 des Patientenventils 3 kommt und dieses dann von der Austrittsöffnung 17 abhebt.

Bei einer in Fig. 4 dargestellten mechanisch arbeitenden Anordnung 27 ist ein Hebel 28 mit der Deckplatte 19 des Beatmungsbeutels 1 verbunden. Sobald das Ende des Kompressionsweges des Beatmungsbeutels erreicht ist, kommt der Hebel 28 in Anlage gegen das verstellbare Teil 16 des Patientenventils 3 und hebt dieses von der Austrittsöffnung 17 ab, so daß auch hier die notwendige Entlastung und damit das Ausatmen des Patienten erreicht wird.

In Fig. 3 und Fig. 4 ist noch schematisch durch einen Pfeil 29 dargestellt, wie durch Komprimieren des Beatmungsbeutels 1 das Einatmen bewirkt wird.

Anhand Fig. 5 werden weitere Einzelheiten näher erläutert.

Der Faltenbalg 20 ist über umlaufende Falze 30 fest und nach außen dicht mit entsprechenden umlaufenden Flanschen 31 der Deckplatte 19 bzw. 32 der Bodenplatte 14 fest verbunden. Die Deckplatte 19 ist im wesentlichen eben ausgebildet, sie kann leicht nach außen gewölbt ausgebildet sein. Die Bodenplatte 14 ist zweckmäßigerweise bodenseitig leicht nach außen gewölbt ausgebildet, sie kann jedoch auch im wesentlichen eben sein. Sie weist wannenartig nach oben ragende Ränder auf, an deren Oberende der Flansch 32 nach außen weist. Hierdurch wird ein umlaufender Ringabschnitt 33 gebildet. In dem umlaufenden Ringabschnitt 33 ist ein Stutzen 34 zum Anschluß der Verbindungsleitung 4 vorgesehen. Ferner ist ein Ansaugventil 35 vorgesehen, über das gemäß dem Pfeil 7 Umgebungsluft angesaugt werden kann. Das Ansaugventil 35 weist in üblicher Weise ein verstellbares Teil 36 wie ein Plättchen, eine Membran oder dergleichen auf, das bei Expansion des Beatmungsbeutels 1 eine Einlaßöffnung 37 freigibt, über die dann die Luft 7 angesaugt wird. Bei der Kompressionsbewegung des Beatmungsbeutels 1 wird dieses verstellbare Teil 36 gegen die Einlaßöffnung 37 gedrückt, so daß Luft im Innenraum des Beatmungsbeutels 1 nur über den Stutzen 34 und die Verbindungsleitung 4 ausströmen kann. Ferner ist, wie dargestellt, zusätzlich ein Sauerstoffanschlußstutzen 38 vorgesehen, über den mittels eines Schlauches Sauerstoff von einer Sauerstoffquelle wie einer Sauerstoffflasche zugeführt werden kann. Sobald die Einlaßöffnung freigegeben ist, wird der Sauerstoff zusammen mit der Umgebungsluft in den Innenraum des Beatmungsbeutels 1 einströmen, der Sauerstoffanteil kann durch Erhöhen des Sauerstoffdruckes erhöht werden. Gegebenenfalls kann es zweckmäßig sein, das verstellbare Teil 36 des Ansaugventils 35 mit Hilfe einer Feder (nicht dargestellt) gegen die Einlaßöffnung 37 vorzuspannen. Gegebenenfalls kann aus Sicherheitsgründen im Stutzen 38 ein Überdruckventil vorgesehen sein.

In vielen Fällen genügt die Eigenelastizität des Faltenbalgs 20, um nach Lösen der Kraft gemäß dem Pfeil 5 eine elastische Expansion sicherzustellen. Wie dargestellt, ist es jedoch zweckmäßig, im Innenraum eine Expansionsfeder 39 vorzusehen. Diese stellt die Expansion sicher und erreicht eine gewisse Führung.

Wie dargestellt, muß das Entlastungsventil 8 nicht notwendigerweise vollständig im Innenraum des Beatmungsbeutels 1 angeordnet sein, wie das in Fig. 2 angedeutet ist. Vielmehr kann es an der Außenseite der Deckplatte 19 aufgesetzt sein. Dargestellt ist eine mit der Deckplatte 19 fest verbundene zylindrische Wanne 40, an deren Boden 41 sich die Feder 11 abstützt und das verstellbare Teil 9 gegen die Öffnung 10 des Entlastungsventils 8 zum Schließen des Entlastungsventils 8 drückt. In den umlaufenden Seitenwänden sind im wesentlichen radial gerichtete Austrittsöffnungen 42 in der Wanne 40 vorgesehen, über die bei freigegebener Öffnung 10 der Druckausgleich entsprechend dem Pfeil 15 gemäß Fig. 2 erfolgen kann. Die in Fig. 5 gezeigte Ausführung hat den Vorteil, daß keine besondere Aufmerksamkeit darauf gerichtet werden muß, an welcher Stelle der Deckplatte 19 der Druck gemäß dem Pfeil 5 ausgeübt wird, die Auslaßöffnung, über die die Entlastung gemäß dem Pfeil 15 bei dem Entlastungsventil 8 erfolgt, kann nicht versehentlich verschlossen werden.

Wie bereits erwähnt, ist ein Widerlager 13 an der Bodenplatte 14 und/oder ein entsprechendes Widerlager 18 an der Deckplatte 19 vorgesehen. Dies hat mehrere Gründe. Zum einen ist es nicht empfehlenswert das Steuerteil 12 besonders lang zu machen, da es das empfindlichste Teil des Entlastungsventils 8 ist und bei zu großer Länge zu leicht abbrechen könnte. Andererseits benötigt der umlaufende Ringabschnitt 33 der Bodenplatte 14 (oder, bei entsprechender Ausbildung, ein entsprechender Ringabschnitt an der Deckplatte 19) eine bestimmte Höhenerstreckung. Schließlich weist der Faltenbalg 20 ebenfalls auch im komprimierten Zustand eine gewisse endliche Höhenerstreckung auf, die durch die Dicke des verwendeten Materials und die Anzahl der Faltungen bedingt ist. Somit ergibt sich, das um eine sichere Anlage des Steuerteils 12 derart zu erreichen, das dieses gegen die Kraft der Feder 11 zum Abheben des verstellbaren Teils 9 bewegt wird, wobei die Höhenerstreckung dieses Steuerteils 12 nicht zu groß sein soll, daß ein entsprechendes Widerlager bestimmter Höhe vorgesehen sein muß.

Beim dargestellten Ausführungsbeispiel gemäß Fig. 5 ist ein einziges Widerlager 13 fest mit der Bodenplatte 14 verbunden. Es ist im wesentlichen topfförmig ausgebildet undweist zahlreiche Perforationen 43 auf. Dies hat den Vorteil, daß bei ständig strömendem Sauerstoff auch im komprimierten Zustand der endliche Totraum innerhalb des Beatmungsbeutels mit Sauerstoff gefüllt werden kann. Dies ist im Gegensatz zu herkömmlichen Anordnungen mit Sauerstoff-Zufuhrstutzen, bei denen nur während des Expansionsvorganges eine entsprechend lediglich geringe Menge mit der Umgebungsluft angesaugt werden kann. Selbstverständlich ist zumindest in dem dem Steuerteil 12 gegenüberliegenden Flächenabschnitt des Bodens des topfförmigen Widerlagers 13 keine entsprechende Öffnung vorgesehen.

Bei der Herzmassage ist es von Vorteil, wenn über den Beatmungsbeutel 1 bzw. deren beiden Platten 14 und 19 ein im wesentlichen vertikaler Druck zur Herzmassage erfolgt, d.h. ein Druck, der im wesentlichen im Balgachse erfolgt. Aus diesem Grund ist es zweckmäßig, eine entsprechende Führungseinrichtung vorzusehen.

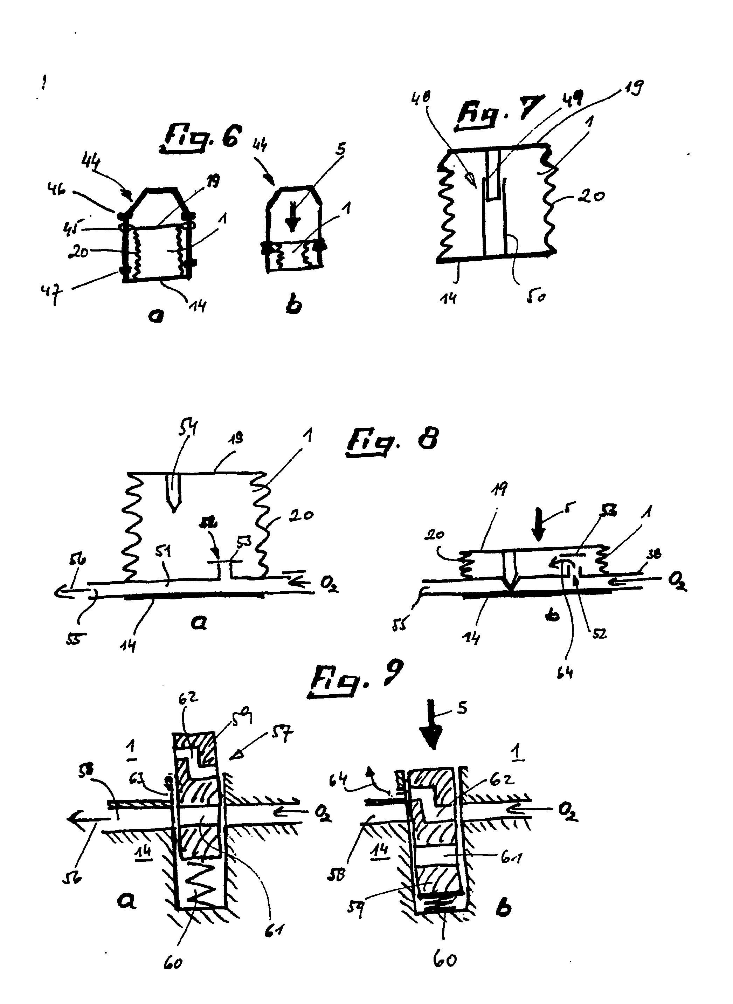

Fig. 6 zeigt schematisch eine Ausführungsform einer solchen Führungseinrichtung. Sie besteht aus einem außenliegenden Rahmen 44 längs dessen zur Balgachse parallelen Stäbe der Balg bzw. dessen Deckplatte 19 über randseitige Ringe 45 oder dergleichen Führungselemente führbar ist. An den Stangen des Rahmens 44 können verstellbar Anschläge 46 und/oder 47 vorgesehen sein, wodurch die Hubhöhe begrenzt werden kann. Der Rahmen kann auch als Trageinrichtung dienen und schützt darüber hinaus den vergleichsweise empfindlichen Faltenbalg 20 des Beatmungsbeutels 1. Die Ringe 45 bzw. die entsprechenden anderen Führungselemente können mit der mechanischen Vorrichtung 27 gemäß Fig. 4 einteilig ausgebildet sein.

Gemäß Fig. 7 kann auch eine teleskopartige Führungseinrichtung vorgesehen sein mit einem Stangenelement 49 und einem Rohrelement 50, die ineinander führbar sind. Solche teleskopartigen Führungseinrichtungen können über den Umfang verteilt sein.

Wie erwähnt, ist es besonders vorteilhaft, wenn reiner Sauerstoff in größerer Menge gesteuert zugeführt werden kann. Da üblicherweise die Sauerstoffquelle ständig zugeschaltet ist, kann es zu Schwierigkeiten kommen. Durch die in Fig. 8 und Fig. 9 dargestellten Ausführungsformen ist es möglich, nur im Komprimierten Zustand, d.h. im wesentlichen im komprimierten Zustand, d.h. im wesentlichen, während der Herzmassage-Zeit den Totraum des Innenraums des Beatmungsbeutels 1 mit reinem Sauerstoff zu füllen und zu allen anderen Zeiten, insbesondere während der Expansionszeit den Sauerstoff ins Freie abzuführen. Ein gesondertes Überdruckventil ist nicht mehr erforderlich. Gemäß Fig. 8 ist ein im wesentlichen elastischer Schlauch an der Innenseite der Bodenplatte 14 vorgesehen, dessen beide Enden nach außen weisen. Der Schlauch 51 weist ein sich durch Überdruck ins Innere des Beatmungsbeutels 1, insbesondere in dessen Totraumbereich, öffendes Überdruckventil 52 auf, das nur schematisch durch das verstellbare Teil 53 wie eine Platte, Scheibe oder dergleichen dargestellt ist. Ferner ist an der gegenüber liegenden Deckplatte 19 ein Druckstempel 54 vorgesehen, der im komprimierten Zustand des Beatmungsbeutels 1 den elastischen Schlauch 51 an der dem Überdruckventil 52 stromabgelegenen Seite abdrückt, wodurch der anstehende Sauerstoff das Überdruckventil 52 öffnet und den Totraum vollständig füllt. Bei der Expansionsbewegung wird der Durchtritt durch den elastischen Schlauch 51 wieder freigegeben, das Überdruckventil 52 schließt und der zugeführte Sauerstoff kann an der anderen Seite über die Austrittsöffnung 55 gemäß dem Pfeil 56 abströmen.

Bei der Ausführungsform gemäß Fig. 9 ist ein Schieberventil 57 vorgesehen, das im Verlauf einer im wesentlichen starren Leitung 58 im Bereich der Bodenplatte 14 vorgesehen ist. Das Schieberteil 59 des Schieberventils 57 wird durch eine Feder 60 in einer Stellung gehalten, in der eine Durchgangsöffnung 61 den Strömungsweg durch die Leitung 58 freigibt, entsprechend dem Pfeil 56. Bei der Kompression gemäß dem Pfeil 5, wird das Schieberteil 59 verschoben, derart, daß eine zweite Durchgangsöffnung 62 stromaufseitig mit der Einströmseite der Leitung 58 in Verbindung kommt, während sich die Stromabseite der Durchgangsöffnung 62 in den Innenraum des Beatmungsbeutels 1 über eine Öffnung 63 öffnet, derart, daß der Sauerstoff gemäß dem Pfeil 64 in den Innenraum bzw. den zur Verfügung stehenden Totraum des Beatmungsbeutels 1 strömen kann. Bei Auslösung der Expansion drückt die Feder 60 das Schieberteil wieder in die in Fig. 9a dargestellte Lage zurück.

Fig. 5a zeigt eine Ausführungsform, bei der Entlastungsventil 8 und Ansaugventil 35 integriert ausgebildet sind. Die Öffnung 10 außerhalb des Bereichs des verstellbaren Teils 9 des Entlastungsventils 8 umgebend sind mehrere Öffnungen 37 in der Deckplatte 19 vorgesehen. Das verstellbare Teil 36 des Ansaugventils 35 ist ringscheibenförmig und hier als elastische Membran ausgebildet, die am Innenumfang die Öffnung 10 umgebend an der Deckplatte 19 befestigt ist, so daß ein Ansaugen gemäß dem Pfeil 7 über die Öffnungen 37 des Ansaugventils 35 erfolgen kann. Die übrigen Einzelheiten sind nicht mehr dargestellt und entsprechen bisher erläuterten.

Selbstverständlich sind noch andere Ausführungsformen möglich, wesentlich ist, daß über eine Entlastungseinrichtung oder dergleichen am Ende des Kompressionsweges eine Druckentlastung derart stattfindet, daß zwangsweise eine Ausatmung über das Patientenventil erreicht wird, ohne daß eine besondere Sorgfalt erforderlich ist, vielmehr kann sofort nach Beendigung des Kompressionsweges die Herzmassage begonnen werden.

## Ansprüche

1. Vorrichtung zur Herzmassage und zur Beatmung, mit einem mit Mund und/oder Nase eines zu beatmenden Patienten verbindbaren Patientenadapter (2), wie Atemmaske oder Intubationsschlauch, mit einem auf dem Brustkorb des zu massierenden Patienten aufzusetzenden komprimierbaren elastisch rück-expandierenden Beatmungsbeutels (1), mit einem dem Beatmungsbeutel (1) zugeordneten Luftansaugventil (35), über das Luft in den Beatmungsbeutel (1) bei dessen Expansion saugbar ist, und mit einem Patientenventil (3) in einer Verbindungsleitung (4) entsprechender Länge zwischen Beatmungsbeutel (1) und Patientenadapter (2) mit einem verstellbaren Teil (16), wie einer Membran, einem Plättchen oder dergleichen, das bei Ausübung der Kompression den Strömungsweg zwischen Beatmungsbeutel (1) und Patientenadapter (2) freigibt und die Auslaßöffnung (17) verschließt und das bei Lösen des Kompressionsdruckes den Strömungsweg zwischen Beatmungsbeutel (1) und Patientenadapter (2) verschließt und die Auslaßöffnung (17) zwecks Ausatmens freigibt, **gekennzeichnet** durch ein normalerweise geschlossenes Entlastungsventil (8), das mit dem Beatmungsbeutel (1) oder der Verbindungsleitung (4) zwischen Beatmungsbeutel (1) und Patientenventil (3) oder dem Patientenventil (3) verbunden ist, und dessen verstellbares Teil (9; 23, 25; 28) ein Steuerteil (12, 22, 26, 28) aufweist, das so in dem Kompressionsbewegungsweg des Beatmungsbeutels (1) angeordnet ist, daß es bei Beendigung des Kompressionsbewegungsweges das verstellbare Teil (9; 16) zwangsweise in die Offenstellung bewegt zur Lösung des Kompressionsdruckes.

2. Vorrichtung nach Anspruch 1, dadurch **gekennzeichnet**, daß der Beatmungsbeutel (1) durch einen zwischen einer steifen Bodenplatte (14) und einer steifen Deckplatte (19) nach außen dicht angeordneten Faltenbalg (20) gebildet ist, wobei zwischen Bodenplatte (14) und Deckplatte (19) eine in Expansionsbewegungsrichtung wirkende Rückstellfeder (39) vorgesehen ist.

3. Vorrichtung nach Anspruch 2, dadurch **gekennzeichnet**, daß an Boden- und/oder Deckplatte (14, 19) steife Widerlager (13, 18) angebracht sind, derart, daß im komprimierten Zustand Deck- und Bodenplatte (14, 19) bzw. deren Widerlager (13, 18) aneinander anliegen.

4. Vorrichtung nach einem der Ansprüche 1 bis 4, dadurch **gekennzeichnet**, daß das Entlastungsventil (8) so in Boden- oder Deckplatte (14, 19) bzw. deren Widerlager (13, 18) angeordnet ist, daß dessen Steuerteil (12) im komprimierten Zustand zur Öffnung des verstellbaren Teils (10) bewegt ist.

5. Vorrichtung nach einem der Ansprüche 1 bis 4, dadurch **gekennzeichnet**, daß eine Bewegung des Steuerteils (22, 28) mechanisch und/oder hydraulisch/pneumatisch auf das verstellbare Teil (16) des Patientenventils (3) zum Freigeben dessen Auslaßöffnung (17) einwirkt.

6. Vorrichtung nach einem der Ansprüche 2 bis 5, **gekennzeichnet** durch eine Führungseinrichtung (44, 45 ; 48), durch die bei der Kompressionsbewegung die Deckplatte (19) im wesentlichen längs der Achse des Beatmungsbeutels (1) zur Bodenplatte (14) bewegbar ist.

7. Vorrichtung nach Anspruch 6, dadurch **gekennzeichnet**, daß die Führungseinrichtung aus mindestens einer im Innenraum des Beatmungsbeutels (1) vorgesehenen Anordnung (48) besteht, bei der ein Stangenteil (49) teleskopartig in ein Führungsrohr (50) eingreift.

8. Vorrichtung nach Anspruch 6, dadurch **gekennzeichnet**, daß die Führungseinrichtung durch eine externe starre Rahmenanordnung (44) gebildet ist, längs der die Deckplatte (19) geführt ist.

9. Vorrichtung nach einem der Ansprüche 6 bis 8, dadurch **gekennzeichnet**, daß die Führungseinrichtung verstellbare Anschläge (46, 47) zur Begrenzung des Hubes, d.h. des Beutelvolumens, im expandierten Zustand aufweist.

10. Vorrichtung nach einem der Ansprüche 1 bis 9, dadurch **gekennzeichnet**, daß das Luftansaugventil (35) und das Entlastungsventil (8) integriert ausgebildet sind (Fig. 5a).

11. Vorrichtung nach einem der Ansprüche 1 bis 9, dadurch **gekennzeichnet**, daß der Beatmungsbeutel (1) einen an eine Sauerstoffquelle wie eine Druckflasche anschließbaren Stutzen (38) aufweist, über den während der Expansionsbewegung zumindest zusätzlich reiner Sauerstoff ansaugbar ist.

12. Vorrichtung nach Anspruch 11 und einem der Ansprüche 2 bis 9, dadurch **gekennzeichnet**, daß der Stutzen (38) so angeordnet ist, daß im komprimierten Zustand des Beatmungsbeutels (1) dessen Toträume mit reinem Sauerstoff füllbar sind.

13. Vorrichtung nach Anspruch 11 oder 12, dadurch **gekennzeichnet**, daß bei ständig angeschlossener Sauerstoffquelle im Innenraum des Beatmungsbeutels (1) ein mit dem Stutzen (38) verbundener elastischer nach außerhalb des Innenraums führender und dort offener Schlauch (51) vorgesehen ist, der ferner ein sich in den Innenraum öffnendes Überdruckventil (52) besitzt, und ein bei der Kompressionsbewegung bewegbares Druckteil (54) vorgesehen ist, das in der Kompressionsstellung den Schlauch (51) zwischen Überdruckventil (52) und Öffnung (55) nach außen abdrückt.

14. Vorrichtung nach Anspruch 11 oder 12, dadurch **gekennzeichnet**, daß bei ständig angeschlossener Sauerstoffquelle im Innenraum des Beatmungsbeutels (1) eine Leitung vorgesehen ist, in deren Verlauf ein Zweistellungs-Schieberventil (57) angeordnet ist, wobei dessen Schieberteil (59) bei der Kompressionsbewegung so bewegbar ist, daß in der Kompressionsstellung Sauerstoff aus der Leitung (58) über das Schieberteil (59) in den Innenraum des Beatmungsbeutels (1) und im übrigen über das Schieberteil (59) und über den anschließenden Leitungsteil nach außen strömt.

15. Vorrichtung nach einem der Ansprüche 10 bis 14 und einem der Ansprüche 3 bis 9, dadurch **gekennzeichnet**, daß das mindestens eine Widerlager (13) topfförmig ausgebildet ist und Perforationen (43) in der Topfwand aufweist zur Erhöhung des Totraumvolumens im Innenraum des Beatmungsbeutels (1).

16. Vorrichtung nach einem der Ansprüche 2 bis 15, dadurch **gekennzeichnet**, daß das Entlastungsventil (8) in der Deckplatte (19) so angeordnet ist, daß dessen mindestens eine Öffnung (42) nach außen eine im wesentlichen radial gerichtete Achse aufweist.

## Claims

1. Apparatus for cardiac massage and respiration comprising a patient adapter (2), such as a respiration mask or intubation hose, which can be connected to the mouth and/or nose of a patient to be given artificial respiration, a compressible, elastically re-expandable respiration bag (1) to be placed on the thorax of the patient to be massaged, an air intake valve associated with the respiration bag (1) through which the air can be sucked into the respiration bag (1) when it expands, and a patient valve (3) in a connection line (4) of suitable length between the respiration bag (1) and the patient adapter (2) having a displaceable part (16) such as a membrane, a small plate or the like, which on compression frees the flow path between the respiration bag (1) and the patient adapter (2) and closes the outlet opening (17) and on releasing the compression pressure blocks the flow path between the respiration bag (1) and the patient adapter (2) and opens the outlet opening (17) for respiration, characterised by a normally closed pressure relief valve (8) that is connected to the respiration bag (1) or to the connecting line (4) between the respiration bag (1) and the patient valve (3) or to the patient valve (3), and of which the displaceable part (9 ; 23, 25 ; 28) has a control part (12, 22, 26, 28) which is arranged in the path of the compression movement of the respiration bag (1) so that on completion of the compression movement the displaceable part (9 ; 16) necessarily moves into the open position to release the compression pressure.

2. Apparatus according to claim 1, characterised in that the respiration bag (1) comprises a bellows (20) arranged sealed to the outside, between a rigid bottom plate (14) and a rigid cover plate (19), with a return spring (39) acting in the direction of the expansion movement being provided between the bottom plate (14) and the cover plate (19).

3. Apparatus according to claim 2, characterised in that rigid abutments (13, 18) are provided on the bottom and/or cover plate (14, 19) so that in the compressed state the cover and bottom plates (14, 19) or their abutments (13, 18) bear on one another.

4. Apparatus according to any one of claims 1 to 4, characterised in that the pressure relief valve (8) is arranged in the bottom or cover plate (14, 19) or their abutments (13, 18) so that when compressed its control part (12) is moved to open the displaceable part (10).

5. Apparatus according to any one of claims 1 to 4, characterised in that a movement of the control part (22, 28) acts mechanically and/or hydraulically/pneumatically on the displaceable part (16) of the patient valve (3) to free its outlet opening (17).

6. Apparatus according to any one of claims 2 to 5, characterised by guide means (44, 45 ; 48) by which, in the compression movement, the cover plate (19) can be moved substantially along the axis of the respiration bag (1) to the bottom plate (14).

7. Apparatus according to claim 6, characterised in that the guide means comprises at least one arrangement (48) provided in the interior of the respiration bag (1), in which a rod part (49) engages telescopically in a guide tube (50).

8. Apparatus according to claim 6, characterised in that the guide means comprises an external rigid frame arrangement (44) along which the cover plate (19) is guided.

9. Apparatus according to any one of claims 6 to 8, characterised in that the guide means has adjustable stops (46, 47) to limit the stroke, i.e. the volume of the bag, in the expanded state.

10. Apparatus according to any one of claims 1 to 9, characterised in that the air intake valve (35) and the pressure relief valve (8) are integrated (Fig. 5a).

11. Apparatus according to any one of claims 1 to 9, characterised in that the respiration bag (1) has connections (38) which can be connected to an oxygen supply such as a gas cylinder, through which at least additional pure oxygen can be sucked in during the expansion movement.

12. Apparatus according to claim 11 and any one of claims 2 to 9, characterised in that the connections (38) are arranged so that when the respiration bag (1) is compressed its dead spaces can be filled with pure oxygen.

13. Apparatus. according to claim 11 or claim 12, characterised in that when the oxygen supply is permanently connected an elastic hose (51) is provided in the interior of the respiration bag (1) that is connected to the connections (38) and leads outside from the interior and is open there, and in addition has an excess pressure valve (52) opening into the interior, and a pressure piece (54) is provided that moves with the compression movement and in the compressed state constricts the hose (51) between the excess pressure valve (52) and the opening (55) to the exterior.

14. Apparatus according to claim 11 or claim 12 characterised in that when the oxygen supply is permanently connected a line is provided in the interior of the respiration bag (1) in which a two-way slide valve (57) is arranged, whereof the slide part (59) is able to move with the compression movement so that in the compressed state oxygen flows out of the line (58) via the slide part (59) into the interior of the respiration bag (1) and also flows outside via the slide part (59) and via the connecting line part.

15. Apparatus according to any one of claims 10 to 14 and any one of claims 3 to 9, characterised in that at least one abutment (13) is cup-shaped and has perforations (43) in the wall of the cup to increase the volume of the dead space in the interior of the respiration bag (1).

16. Apparatus according to any one of claims 2 to 15, characterised in that the pressure relief valve (8) is arranged in the cover plate (19) so that at least one opening (42) thereof has an axis that is directed substantially radially.

**Revendications**

1. Dispositif pour le massage cardiaque et pour la respiration assistée au moyen d'un élément (2) prévu pour l'adaptation au patient susceptible d'être relié à la bouche ou au nez d'un patient dont la respiration doit être assistée, comme par exemple un masque respiratoire ou une sonde d'intubation, comportant un sac respiratoire (1) qui doit être mis en place sur la cage thoracique du patient qu'il s'agit de masser, susceptible d'être comprimé et de se réexpanser élastiquement, comportant une valve (35) d'aspiration d'air affectée au sac respiratoire (1), par l'intermédiaire de laquelle l'air peut être aspiré dans le sac respiratoire (1) lors de l'expansion de celui-ci, et comportant une valve (3) destinée au patient dans un conduit de liaison (4) de longueur correspondante entre le sac respiratoire (1) et l'élément (2) prévu pour l'adaptation au patient, munie d'une pièce réglable (16), telle qu'une membrane, une plaquette ou une pièce similaire, qui, lorsque l'on exerce la compression, libère le passage d'écoulement entre le sac respiratoire (1) et l'élément (2) prévu pour l'adaptation au patient et ferme l'orifice de sortie (17), et qui, lors du relâchement de la pression de compression, forme le passage d'écoulement entre le sac respiratoire (1) et l'élément (2) prévu pour l'adaptation au patient, et qui libère l'orifice de sortie (17) en vue de l'expiration, caractérisé par une valve (8) de soulagement normalement fermée qui est reliée au sac respiratoire (1) ou au conduit de liaison (4) entre le sac respiratoire (1) et la valve (3) du patient, ou à la valve (3) du patient, et dont la pièce réglable (9 ; 23, 25 ; 28) présente une pièce de commande (12, 22, 26, 28), qui est ainsi disposée dans le chemin de déplacement de compression du sac respiratoire (1), qu'à la fin du chemin de déplacement de compression elle déplace la pièce réglable (9 ; 16) de force en position d'ouverture à la fin du chemin de déplacement de compression, en vue de relâcher la la pression de compression.

2. Dispositif selon la revendication 1, caractérisé par le fait que le sac respiratoire (1) est formé par un soufflet (20) disposé de façon étanche par rapport à l'extérieur, entre une plaque de base rigide (14) et une plaque de couverture rigide (19), un ressort de rappel (39) agissant dans le sens du déplacement d'expansion étant prévu entre la plaque de base (14) et la plaque de couverture (19).

3. Dispositif selon la revendication 2, caractérisé par le fait que des butées (13, 18) rigides sont disposées sur la plaque de base et/ou la plaque de couverture (14, 19), de façon telle qu'à l'état comprimé la plaque de couverture et la plaque de base (14, 19) c'est-à-dire leurs butées (13, 18) reposent l'une sur l'autre.

4. Dispositif selon l'une des revendications 1 à 4, caractérisé par le fait que la valve de soulagement (8) est disposée dans la plaque de base, ou dans la plaque de couverture (14, 19), respectivement dans leurs butées (13, 18) de façon telle que sa pièce de commande (12) est déplacée à l'état comprimé en vue de l'ouverture de la pièce réglable (10).

5. Dispositif selon l'une des revendications 1 à 4, caractérisé par le fait qu' un déplacement de la pièce de commande (22, 28) a une action mécanique et/ou hydraulique, pneumatique sur la pièce réglable (16) de la valve (3) destinée au patient en vue de libérer l'orifice de sortie (17) de celle-ci.

6. Dispositif selon l'une des revendications 2 à 5, caractérisé par un dispositif de guidage (44, 45 ; 48) grâce auquel lors du déplacement de compression la plaque de couverture (19) est susceptible d'être déplacée sensiblement le long de l'axe du sac respiratoire (1) en direction de la plaque de fond (14).

7. Dispositif selon la revendication 6, caractérisé par le fait que le dispositif de guidage est constitué par au moins un agencement prévu dans l'espace intérieur du sac respiratoire (1), dans lequel une pièce (49) en forme de tige pénètre dans un tube de guidage (50) de façon télescopique.

8. Dispositif selon la revendication 6, caractérisé par le fait que le dispositif de guidage est constitué par un dispositif externe fixe en forme de cadre (44) le long duquel est guidée la plaque de couverture (19).

9. Dispositif selon l'une des revendications 6 à 8, caractérisé par le fait que le dispositif de guidage présente à l'état expansé des butées réglables (46, 47) destinées à limiter la course c'est-à-dire le volume du sac.

10. Dispositif selon l'une des revendications 1 à 9, caractérisé par le fait que la valve (35) d'aspiration d'air et la valve (8) de soulagement, sont réalisées de façon à former une seule pièce (figure 5a).

11. Dispositif selon l'une des revendications 1 à 9, caractérisé par le fait que le sac respiratoire (1) présente un embout susceptible d'être raccordé à une source d'oxygène, telle qu'une bouteille sous pression par l'intermédiaire duquel de l'oxygène pur peut être aspiré, au moins en complément, pendant le déplacement d'expansion.

12. Dispositif selon la revendication 11 et l'une des revendivations 2 à 9, caractérisé par le fait que l'embout (38) est disposé de façon telle que le sac respiratoire (1) étant à l'état comprimé ses espaces morts peuvent être remplis d'oxygène pur.

13. Dispositif selon la revendication 11 ou 12, caractérisé par le fait que la source d'oxygène étant raccordée en permanence, on prévoit dans l'espace intérieur du sac respiratoire (1), un tuyau élastique relié à l'embout (38), conduisant vers l'extérieur de l'espace intérieur, et ouvert à cet endroit, qui possède de plus une valve de surpression (52) ouvrant l'espace intérieur, et que l'on prévoit une pièce de compression (54) susceptible d'être déplacée lors du mouvement de compression, qui, en position de compression repousse vers l'extérieur le tuyau (51) entre la valve de surpression (52) et l'ouverture (55).

14. Dispositif selon la revendication 11 ou 12, caractérisé par le fait que la source d'oxygène étant raccordée en permanence, on prévoit dans l'espace intérieur du sac respiratoire (1) un conduit dans le parcours duquel est disposée une valve (57) à tiroir comportant deux positions, la partie (59) formant tiroir de celle-ci étant susceptible d'être déplacée de façon telle lors du mouvement de compression, qu'en position de compression l'oxygène s'écoule du conduit (58) par l'intermédiaire de la pièce (59) formant tiroir, dans l'espace intérieur du sac respiratoire (1), et en outre vers l'extérieur par l'intermédiaire de la pièce (59) formant tiroir et par l'intermédiaire de la pièce de conduit qui lui est raccordée.

15. Dispositif selon l'une des revendications 10 à 14 et l'une des revendications 3 à 9, caractérisé par le fait qu' au moins une butée (13) a une forme de cuvette et présente des perforations (43) dans la paroi de la cuvette, en vue d'augmenter le volume d'espace mort dans l'espace intérieur du sac respiratoire (1).

16. Dispositif selon l'une des revendications 2 à 15, caractérisé par le fait que la valve de soulagement (8) est disposée dans la plaque de couverture (19) de façon telle que son ouverture au minimum unique (42) présente vers l'extérieur un axe dirigé sensiblement radialement.

Fig. 1

Fig. 2

Fig. 3

Fig. 4

Fig. 5

Fig. 5a

Fig. 6

Fig. 7

Fig. 8

Fig. 9